(19)

**Europäisches Patentamt**
**European Patent Office**
**Office européen des brevets**

(11) **EP 4 193 923 A1**

(12) **EUROPEAN PATENT APPLICATION**

(43) Date of publication:
**14.06.2023  Bulletin 2023/24**

(21) Application number: **21212800.3**

(22) Date of filing: **07.12.2021**

(51) International Patent Classification (IPC):
*A61B 5/113* (2006.01)   *A61B 5/091* (2006.01)
*A61B 5/08* (2006.01)   *A61B 5/11* (2006.01)
*A61B 5/0507* (2021.01)

(52) Cooperative Patent Classification (CPC):
**A61B 5/1135; A61B 5/091; A61B 5/1128;**
A61B 5/0077; A61B 5/0507; A61B 5/08;
A61B 5/0826; A61B 5/1118; A61B 5/4561

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **KATIPALLY, Karthik Raj**
**Eindhoven (NL)**

• **BERA, Deep**
**Eindhoven (NL)**
• **CHAKRABARTI, Biswaroop**
**Eindhoven (NL)**
• **RADHAKRISHNAN, Ravindranath**
**Eindhoven (NL)**
• **HIWALE, Sujitkumar**
**Eindhoven (NL)**

(74) Representative: **Philips Intellectual Property &**
**Standards**
**High Tech Campus 52**
**5656 AG Eindhoven (NL)**

(54) **A SYSTEM AND METHOD FOR REMOTE MONITORING OF RESPIRATION OF SUBJECT**

(57)     A system for remote monitoring of respiration of subject processes images and depth information in respect of a field of view. For subjects in the field of view, a body posture is determined and when the subject body posture is suitable, respiratory parameters indicative of respiratory effort are measured using temporal analysis of the images and depth information in respect of a chest area of the subject.

FIG. 2

EP 4 193 923 A1

## Description

FIELD OF THE INVENTION

This invention relates to the monitoring of respiration of a subject, for example for monitoring a subject with a chronic respiratory disease.

BACKGROUND OF THE INVENTION

Chronic respiratory diseases (CRDs) are diseases of the airways and other structures of the lung. Some of the most common are asthma, chronic obstructive pulmonary disease (COPD), occupational lung diseases and pulmonary hypertension. In addition to tobacco smoke, other risk factors include air pollution, occupational chemicals and dusts, and frequent lower respiratory infections during childhood.

CRDs are not curable. However, various forms of treatment that help dilate major air passages and improve shortness of breath can help control symptoms and increase the quality of life for people with the disease. For example, inhalers are used for clearing airways in the lungs, and air purifiers are used for filtering pollutants which have adverse effects on COPD patients.

COPD is a group of progressive lung disorders characterized by long-term breathing problems and poor airflow. The main symptoms include shortness of breath and cough with sputum production. According to World Health Organization estimates, 65 million people have moderate to severe chronic obstructive pulmonary disease (COPD).

A COPD grade is a severity measure of COPD and is usually done with a spirometry test. Spirometry is a gold standard measure for a pulmonary function test. It is usually a one-time test and the grading will be based on forced expiration action by the patient indicative of lung capacity.

Current measurements of respiratory parameters require patients to follow a sequence of instructions for evaluation, and most of these solutions are contact-based solutions. These require patient's attention, cooperation and a portion of their time for evaluation purposes. This is also arduous and inconvenient for a few patients given their profession, disabilities and other factors or medical conditions.

For example, forced expiratory maneuvers may aggravate some medical conditions. Spirometry for example should not be performed when the individual presents with: hemoptysis of unknown origin; pneumothorax; unstable cardiovascular status (angina, recent myocardial infarction, etc.); thoracic, abdominal, or cerebral aneurysms; cataracts or recent eye surgery; recent thoracic or abdominal surgery; nausea, vomiting, or acute illness; recent or current viral infection; or undiagnosed hypertension,

There are also cases where patients with respiratory conditions need to be under constant monitoring for deterioration. Current solutions are either a one-time evaluation like spirometry or need the patient to be tethered or to stay still in a required position or stance. Thus, they are not appropriate for continuous monitoring purposes. Additionally, one-time evaluations are appropriate for only a few patients with mild symptoms.

Plethysmography is a known alternative to spirometry and provides a functionally equivalent measure. It can capture more information than a spirometry measure. Pulmonary plethysmographs are commonly used to measure the functional residual capacity (FRC) and they are known to correlate with spirometry measures. Plethysmography can be implemented using optoelectronic plethysmography, structured light plethysmography and Kinect (Trader Mark) camera time of flight plethysmography. These methods obtain information about chest wall dynamics.

Optoelectronic plethysmography is invasive, relatively expensive, and inconvenient. Structured light plethysmography is non-invasive and convenient for patients but moderately expensive and not sufficiently portable to be used for monitoring at home. Kinect (Trade Mark) camera plethysmography is non-contact, non-invasive, inexpensive and convenient, but needs patients to be sitting on a chair for a one-time evaluation. Also patients are known to exhibit an observer effect and fail to follow instructions properly.

There is therefore a need for an improved remote monitoring system which can perform repeated measurements, is low cost, quick to use, suitable for people with disabilities, simple to use for the patient (avoiding the need for patient's attention and cooperation) and avoids observer effects in the measurements.

SUMMARY OF THE INVENTION

The invention is defined by the claims.

According to examples in accordance with an aspect of the invention, there is provided a system for remote monitoring of respiration of subject, comprising:

an imaging system comprising an imaging sensor and a time of flight depth sensor; and
a processor for processing the image sensor signals and the depth sensor signals, wherein the processor is adapted to:
receive data comprising images and depth information in respect of a field of view;
process at least part of the data to detect a subject within the field of view;
process at least part of the data to determine a body posture of the subject;
determine when the subject body posture is suitable for the measurement of respiratory parameters, and when it is determined that a subject body posture is suitable for the measurement of respiratory parameters, measure respiratory parameters indicative of respiratory effort using temporal analysis of the images and depth information in respect of a chest area of the subject.

The analysis implements remote plethysmography of the chest area, so that respiratory parameters may be obtained. The system may be used to monitor patients with respiratory conditions such as COPD and asthma, and can play an important role in improving their quality of life.

The system can be implemented as a non-contact based monitoring system. The respiratory parameters for example provide a surrogate measure of tidal volume. The system can be passive, instruction free, and may provide a home-based continuous monitoring solution.

The posture detection can be estimated and classified using a skeleton tracking system, such as the Kinect (Trade Mark) skeleton tracking system using depth camera information. The nodes of the skeleton data can be used to detect body posture, for example so that it can be classified if the subject is front-facing or not. Using skeleton data is efficient, for example with monitoring of only 14 nodes. To improve the accuracy, sensor image data can additionally be used to collect data and train a neural network to classify if the subject body posture is suitable (e.g. the subject is front-facing).

Deep learning models can also provide posture detection from regular RGB cameras without depth information.

The temporal analysis of depth data in combination with the image data (so that it is known which anatomical areas are associated with the depth data) performs the remote plethysmography by analyzing chest motion.

The processor may be adapted to detect a front-facing subject orientation with respect to the imaging system.

This enables chest motion to be captured. The detailed analysis can be inhibited when the subject is not facing the imaging system.

The processor may be adapted to identify anatomical landmarks of the chest.

The localizing of the landmark points on the chest area for example differentiates the thoracic cage based on the upper, middle and lower compartmental lobes. This is equivalent to a pose detection but a with restriction to only the chest area combined with classification of compartmental lobes. Continuous tracking of these landmark points will allow the subject to move untethered in front of the imaging system and the overall system will be able to account for movement while taking measurements.

The processor may be adapted to collect depth data for analysis of chest wall dynamics. The depth data enables movement information to be obtained of the chest lobes.

The processor may be adapted to determine a tidal volume for different chest compartments. The tidal volume is of particular interest for assessing a state of respiratory conditions such as COPD.

The processor may be adapted to determine a tidal volume based on temporal amplitude and phase differences between time of flight measurements for different chest compartments.

The processor is for example adapted to perform activity detection to detect a level of activity of the subject.

In this way, measurements may be taken only when the subject has a particular level of activity, such as being at rest or with an activity level below a threshold.

The activity recognition can also be fused with a skeleton detection system to improve robustness. The activity of the subject may for example be classified into three bins (i) Low (ii) Medium and (iii) High. Activity classification can be simply done using temporal data of a fused image segmentation output and skeleton detection system output.

The imaging system may comprise a plurality of types of time of flight sensor.

This enables the system to function in different conditions and ranges. For example, the system may use ultrasound, Terahertz and/or mmWave frequencies, in addition to visible light imaging.

The imaging system for example comprises a RGB sensor for use in detecting a subject within the field of view and determining the body posture and a radar or ultrasound radar sensor for use in identifying and tracking anatomical landmarks of the chest.

The radar or ultrasound sensor (for example a mmWave radar sensor e.g. 30GHz to 300GHz) enables the system to cope with loose clothing, and loose clothing classification logic may be used. The radar or ultrasound sensor has a longer range capability and is penetrable through clothing. This means a range estimation can be obtained which encodes purely chest wall dynamics. The image sensor is for example used to localize landmarks on the chest area and adaptive beam forming using the radar or ultrasound sensor may then be used to obtain the range data.

The processor may be adapted to choose different sensor types based on a distance to the subject.

The processor is for example adapted to derive a respiratory minute volume from a continuously estimated tidal volume.

The invention also provides a method for remote monitoring of respiration of subject, comprising:

receiving data comprising images and depth information in respect of a field of view;
processing at least part of the data to detect a subject within the field of view;
processing at least part of the data to determine a body posture of the subject;
determining when a subject body posture is suitable for the measurement of respiratory parameters, and when it is determined that the subject body posture is suitable for the measurement of respiratory parameters, measuring respiratory parameters indicative of respiratory effort using temporal analysis of the images and depth information in respect of a chest area of the subject.

The method may a non-contact and remote method for monitoring patients with respiratory conditions such as COPD and asthma.

The method may comprise:

detecting a front-facing subject orientation with respect to the imaging system.
identifying anatomical landmarks of the chest;
collecting depth data for analysis of chest wall dynamics; and
determining a tidal volume for different chest compartments.

The method may comprise performing activity detection to detect a level of activity of the subject and measuring respiratory parameters only for activity levels meeting activity level criteria.

The invention also provides a computer program comprising computer program code means which is adapted, when said program is run on a computer, to implement the method defined above.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Figure 1 shows a system for remote monitoring of respiration of subject;
Figure 2 shows a method for remote monitoring of respiration of subject; and
Figure 3 shows the steps for landmark localization addressing the scenario of loose clothing.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a system for remote monitoring of respiration of subject. The system processes images and depth information in respect of a field of view. For subjects in the field of view, a body posture is determined and when the subject body posture is suitable, respiratory parameters indicative of respiratory effort are measured using temporal analysis of the images and depth information in respect of a chest area of the subject.

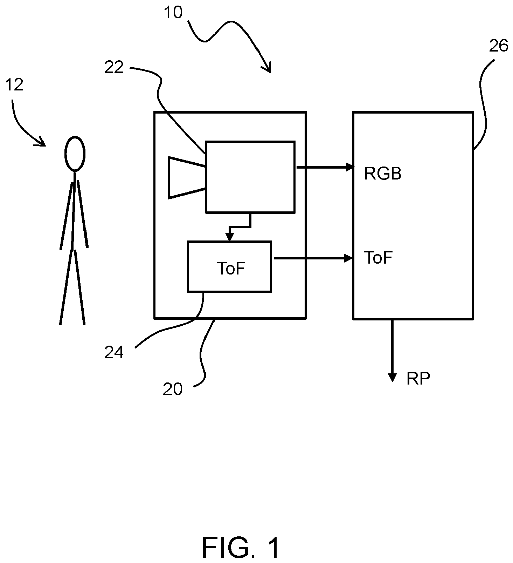

Figure 1 shows a system 10 for remote monitoring of respiration of subject 12. The system comprises an imaging system 20 having an imaging sensor 22 (e.g. an RGB image sensor) and a time of flight depth sensor 24. A processor 26 processes the image sensor signals and the depth sensor signals. The processor receive data comprising images RGB and depth information ToF in respect of a field of view. The data (or part of it) is processed to detect a subject 12 within the field of view and to determine a body posture of the subject 12.

It is then determined if the subject body posture is suitable for the measurement of respiratory parameters, and when it is determined that a subject body posture is suitable for the measurement of respiratory parameters, the respiratory parameters are measured. These parameters are indicative of respiratory effort, and they are obtained using temporal analysis of the images and depth information in respect of a chest area of the subject.

The system may optionally also perform activity detection to detect a level of activity of the subject. Thus, respiratory parameters may be measured only for activity levels meeting activity level criteria, for example for subjects substantially at rest. This ensures that successive measures relate to the same state of exertion of the subject.

The analysis thereby implements remote plethysmography of the chest area, so that respiratory parameters may be obtained. The system may be used to monitor patients with respiratory conditions such as COPD and asthma, and can play an important role in improving their quality of life.

The system can be implemented as a non-contact based monitoring system. The respiratory parameters for example provide a surrogate measure of tidal volume. The system can be passive, instruction free, and may provide a home-based continuous monitoring solution.

The system may for example obtain compartmental tidal volume measures, and may be used to evaluate respiratory parameters from untethered, low activity subjects that are able to move while the remote analysis takes place. The subjects may be located at some distance from the imaging system, for example within a range of 8 to 10 meters. The imaging system 20 for example uses the Kinect (Trade Mark) sensor of Microsoft (Trade Mark), and it may additionally use mm wave, Terahertz, or ultrasonic sensing modalities. The system may also switch between different sensing modalities based on the distance of the subject and the range capabilities of the sensors.

A deep learning algorithm may be used for activity detection of the subject and localization of landmarks on the chest. The abdomen may then be segmented as a graph structure.

Temporal analysis of the graph structure and nodes can then be used to estimate the respiratory effort and other respiratory features. Additionally, the status of the respiratory parameters are recorded whenever the patients' conditions are favorable for the measurement without any special attention and cooperation from them.

In more detail, the depth measurements of several landmarks are obtained for three compartmental lobes of the thoracic cage: the upper, middle and lower lobes, situated on the chest wall, for a given respiratory phase (inspiration or expiration). These landmarks are connected to create a mesh. This mesh is then used to model the chest wall surface and lung volume. Once the surface and volume is reconstructed using the measured depths, a standard 3D motion capture algorithm can be used to track the change of this surface.

During inspiratory efforts, the horizontal section of the rib cage becomes more elliptical whereas during expiratory efforts it becomes more circular. This is explained in more detail in Agostoni E, Mognoni P. "Deformation of the chest wall during breathing efforts", J. Appl. Physiol. 1966 Nov 21(6): 1827-32.

Thereby, the respiratory efforts can be extracted by measuring the change in the volumes of compartmental lobes.

This approach is valid for the loose clothing scenario where the depth maps are extracted using mm Wave or Ultrasound sensors. For tight clothing the depth map creation may use landmark localization (using e.g. a HRNet model), followed by tracking the landmarks through correspondence during low activity, and depth map generation (using e.g. a UNet model).

The recorded respiratory features may be used for short-term prediction of exacerbation, which can be used to guide related interventions. In an example, the measured and monitored respiratory features can be used as feedback information during rehabilitation and strength-training.

By enabling the respiratory parameters to be measured from low activity subjects, multiple measurements may be taken over suitable durations without requiring the subject's attention or cooperation. By making multiple evaluations, outliers can be removed leading to reduced error measurements. This also removes observer effect from the subjects during evaluation.

The system may be used to monitor patients with COPD, asthma and other respiratory conditions, and can play an important role in improving their quality of life. The system may be implemented as a passive, instruction free, home based continuous monitoring solution. The ability to provide continuous monitoring of respiratory conditions enables exacerbations to be predicted based, or anomalies or deviations from a healthy pattern to be identified, or constant deteriorations over time to be monitored.

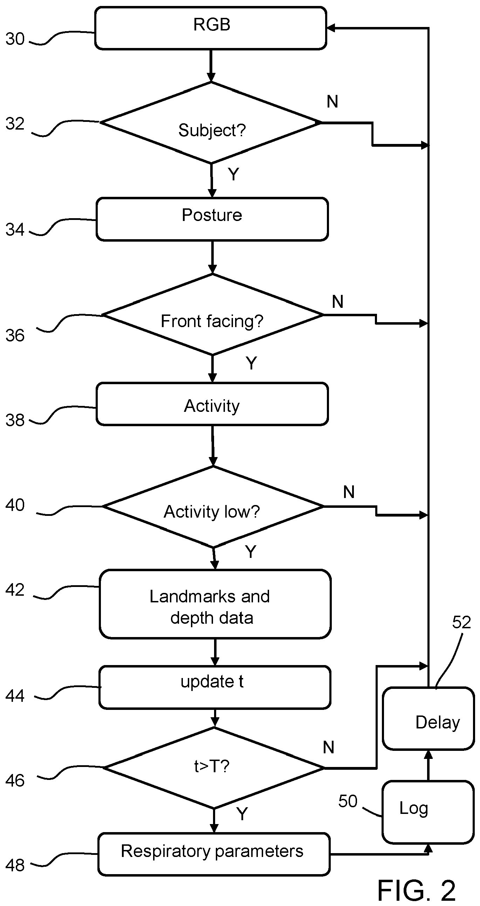

Figure 2 shows a method for remote monitoring of respiration of subject.

In step 30 images are received in respect of a field of view, such as RGB camera images.

In step 32, the images are processed to detect a subject within the field of view. In step 34, the images are processed to determine a body posture of the subject. There may be multiple subjects in the field of view, and in this case the system may identify the subjects before the subsequent steps, so a user can identify the correct subject to be monitored. Alternatively, multiple subjects may be monitored.

In step 36 it is determined when the subject body posture is suitable for the measurement of respiratory parameters. A front-facing subject is suitable for such measurements. Partial measurements may also be obtained if the subject is not front facing. If the body posture is suitable for measurements (or partial measurements), the method continues, but if not, the method returns to the start.

In step 38 an activity level of the subject is determined. In step 40 it is determined if the activity level is low such that respiratory measurements can be made. If so, the method continues, but if not, the method returns to the start.

In step 42, landmarks are identified of the chest area, and depth data is obtained. The depth data will typically already be received in step 30 together with the image data. However the subject detection, posture and activity level detection may use image data alone (hence depth data is not needed until step 42), or else one or more of these steps may use both image data and depth data (in which case the depth data is already received in step 30).

A timer is updated in step 44, which records the amount of data that has been collected. In step 46, while there is insufficient data for respiratory measurements (shown as a time period T), the method returns to the start. Once sufficient data has been collected as determined by the timer, the respiratory parameters are measured in step 48. This involves measuring respiratory parameters indicative of respiratory effort using temporal analysis of the images and depth information in respect of the chest area of the subject, as outlined above.

The measurements are then logged in step 50 and a delay is provided in step 52 before the method starts again.

The respiratory measurements are made by tracking the chest compartments and acquiring depth data for chest wall dynamics. The subject detection, posture detection and (optional) activity level analysis provides added intelligence and robustness such that those chest compartment landmarks can be measured without needing the user's attention.

The steps shown in Figure 2 will now be described in more detail.

The first step is to detect the subject of interest in the field of view of camera. This process of subject detection and recognition is well known and off-the-shelf algorithms can be used, although custom algorithms can also be used for fine tuning of performance. The next step is detecting if the subject of interest is in the front-facing posture because the landmarks on the chest area are needed.

The subject detection and body posture detection for example uses an implicit representation model. This is a computational algorithm which can implicitly model the human body and can track skeletal motions and geometry deformations. These models have a potential to enable continuous monitoring of subjects, their activities and posture. The particular ability that is of interest in this case is an ability to track the human body with geometry and further classify a front-facing geometry of from a back facing geometry, and to distinguish between low activity and high activity.

There is also huge progress in correspondence matching algorithms. These correspondence matching algorithms can match positions of landmarks across different postures. This ability will help track landmarks and their features such as the range estimation of chest wall dynamics as used in this application. These algorithms enable instruction free, pose free measurements effectively removing observer effect. This will also enable taking multiple respiratory measurements continuously effectively.

By way of example, the posture detection can be implemented by using skeleton detection and using a depth mesh, with an intelligent thresholding algorithm or with saliency mapping from the depth camera. It can be assessed whether the subject is front-facing by computing Eigenvalues from a low-poly depth mesh. A low-poly depth mesh is enough to classify a front-facing low mesh from a back facing mesh.

Low-poly mesh evaluation with reasonable resolution enables faster computation of Eigenvalues without loss of accuracy. Posture detection can for example be estimated and classified using skeleton data from the Kinect (Trade Mark) system directly. The nodes of the skeleton data have enough information to classify if the subject is front-facing or not. Using skeleton data is efficient for the number of data points needed, such as 14 nodes. Skeleton data can however be noisy but this can be improved by additionally using the camera feed to collect data and train a neural network to classify if the subject is front-facing or not. A simple thresholding can create a binary classification of front-facing or not.

For the activity classification, activity recognition can be fused with the Kinect (Trade Mark) skeleton to make it robust. The activity of the subject may for example be classified into a number of bins, such as three bins;:(i) Low; (ii) Medium; and (iii) High. The activity level classification can be simply done on temporal data of a fused segmentation output and Kinect (Trade Mark) output.

Once the activity recognition is classified as Low, it is feasible to have localization of landmarks on the chest area and to obtain compartmental landmark classification from fused sensor data. The system can measure minor chest wall excursions in millimeter ranges due to thoracic changes during inspiration and expiration across the three lung lobes on both sides.

A first possible implementation will now be described in greater detail.

The localization of landmarks on the chest area is used to enable temporal analysis using off-the-shelf time of flight cameras and image sensors, using a sensor fusion strategy. The processing is for example performed by an edge computing device, namely a device using a distributed computing paradigm that brings computation and data storage closer to the location where it is needed, to improve response times and save bandwidth. The recorded data and computation takes place on the edge computing device and only feature vectors after computation can be shared to remote end saving bandwidth and protecting privacy of patients.

The objective of the landmark localization is to localize the landmark points on the chest area, in particular covering the lobes of the thoracic cage, and differentiating them as the upper, middle and lower compartmental lobes. This is also equivalent to pose detection with a restriction to only consider the chest area combined with classification of the compartmental lobes. Continuous tracking of these landmark points will allow the subject to move untethered in front of the imaging system, and the processor will be able to account for movement while taking measurements.

Training and using the landmark localization for example involves the following steps:

Training the model

Step 1: Obtain the RGB images and depth date for the front-facing (and low activity) subject. The chest area may be visible or non-visible, for example also allowing loose clothing.

Step 2: Annotate landmarks on the chest area for a given frame if the chest area is visible. If the chest area is not visible, the data points should all point to a null value. This is repeated for successive frames.

Step 3: Design a data-loader with raw frames as input and the 2D landmark locations as the ground truth, splitting the data into test, train and validation.

Step 4: Design an end-to-end trainable pose-detection model such as HRnet (e.g. Ke Sun and Bin Xiao and Dong Liu and Jingdong WangDeep "High-Resolution Representation Learning for Human Pose Estimation" (arxiv1), (arxiv2) CVPR 2019). This enables detection of landmarks.

The deep learning model is trained using a training strategy. Different criteria may be used for ending the training, such as a maximum number of iterations, improvements on a loss function during the last 10 iterations etc. The training strategy also includes class balancing to assure a balanced number of images and examples of different classes.

Step 5: Test the developed model and evaluate if accuracy is within acceptable range.

Use of the model

Step 6: Use the developed model to obtain landmarks on the chest area of a subject breathing.

Step 7: Analyze the depth range estimates of the landmarks temporally from fused depth maps.

Step 8: Check for offsets across lobes of the lungs at both left and right sides of the chest area for additional information to differentiate and detect anomalies.

Step 9: Estimate the tidal volume from these temporal measurements. This approach is for example known from Aoki, H., Miyazaki, M., Nakamura, H., Furukawa, R., Sagawa, R., and Kawasaki, H. (2012). "Noncontact respiration measurement using structured light 3-D sensor," in Paper presented at: SICE Annual Conference (SICE) (Akita).

Step 10: Make a log of this information on a database for temporal analysis across days (or any other interval) for short term exacerbation prediction. A simple regression curve trend analysis with a threshold for a level of deterioration in a unit of time (e.g. a day) may be used to determine when intervention is needed.

The deep learning model described above may operate on each frame independently. This may lead to unstable landmark localizations temporally. Additional graph neural network layers may be used on top of a traditional end-to-end deep learning model to make sure that the temporal distance between the localization points is minimal and smooth. This may be used to make the temporal evolution data more precise and hence the end evaluation will be more accurate. This also makes the landmark localizations robust to occlusions temporally.

This approach involves modifying step 4 described above to add additional graph neural network layers to stabilize the ground truth data points temporally. A temporal distance loss function is for example defined as:

$$temporal\ loss = \sum_{1}^{n} (x_t - x_{t-1})^2$$

This temporal loss measure indicates when there are large changes in data points $x_t$ over a set n of successive data points.

Step 7 described above is then modified to skip sections where there are sudden jumps in temporal data as indicated by the temporal loss value exceeding a threshold.

The collected temporal information can be used as a feature vector for downstream applications, for example to help patients with COPD conditions during rehabilitation activities and strength training. Rehabilitation activities are recommended for COPD patients but not monitored and checked for adherence quantitatively. Compartmental analysis of chest region during breathing exercises and other rehabilitation activities can enable progress to be quantified and enable exacerbation to be predicted using daily log information. The information may also be used for sports activity monitoring for performance optimization purposes.

As mentioned above, the system is preferably able to operate with the subject wearing loose clothing. For this purpose, additional loose clothing classification logic may be used. For example, a radar or ultrasound radar sensor, such as a mm Wave sensor, may be used for identifying and tracking anatomical landmarks of the chest. Radar or ultrasound sensors have a longer range capability and are penetrable through clothing. The resulting range estimation will thereby be purely related to chest wall dynamics.

Although standard pose estimation models can perform well on camera images for tight clothing, subjects are not always expected to wear tight clothing. Thereby, an approach is needed for loose clothing scenarios.

In a loose clothing scenario, mmWave or ultrasound may be used to detect the movements beneath the clothing. To perform this switch, logic is needed to detect the loose clothing scenario.

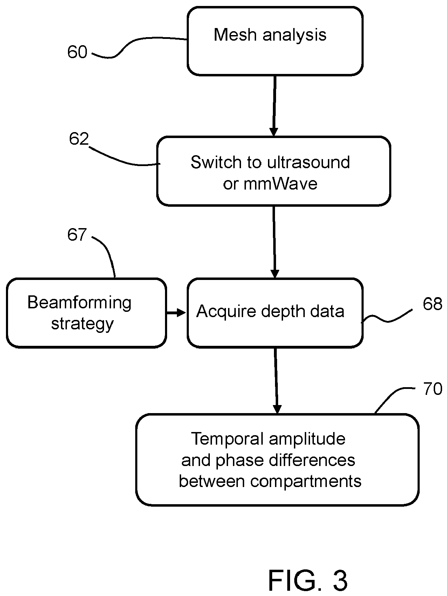

Figure 3 shows the steps for landmark localization addressing the scenario of loose clothing.

In step 60, movements of the points on the mesh are analyzed. For a loose clothing scenario the movement of the points of the mesh on the cloth will be different than for tight clothing. Additionally, there will be an effect of gravity visible on a mesh at the surface of loose clothing. Thus, analysis of the evolution of the mesh over time provides one way to detect loose clothing. The movements of mesh points are classified based on a physics-based and gravity-based model for loose clothing detection.

In step 62, a switch is made to ultrasound or mmWave imaging when loose clothing is detected. The switch to mmWave or ultrasound will effectively make the clothing transparent and will thereby enable accurate measurements of the chest volume detection.

The landmark localization (step 6 above) may be modified to take account of loose clothing detection using reflection spectral analysis using the mmWave or ultrasound imaging.

For the image analysis using the mmWave (radar) or ultrasound, a multiple transmit antenna is used, with a beamforming strategy set in step 67, so that depth data is acquired in step 68 using beam steering to the landmark localizations.

As described above, the respiratory parameters are obtained in step 70 by analyzing temporal amplitude and phase differences between lung lobe compartments.

When the system has multiple sensing modalities, if the subject is beyond the reasonable range of a sensor then it is possible to switch to a different modality.

After localizing the subject and chest area, for each sensor in the set of sensors (Kinect (Trade Mark), mmWave, ultrasonic, etc.) a check can then be made if the subject is in the respective ranges, and the most appropriate sensor is picked using a basic algebraic comparison of ranges. This may be repeated whenever the target reaches the threshold range boundary of the current sensor modality, and the new appropriate sensor modality is then chosen.

As mentioned above, various respiratory parameters may be obtained. One example is to estimate the minute volume, in order to detect silent hypoxia. The "respiratory minute volume" or "minute volume" is the volume of gas inhaled (inhaled minute volume) or exhaled (exhaled minute volume) from a subject's lungs per minute. This measure increases if a subject is unable to maintain sufficient oxygen saturation the blood and tries (often without awareness of it) to address the issue by increasing the breath rate and/or tidal volume. This is often observed when a disease condition (e.g. Covid-19) is responsible for development of hypoxia. As the oxygen saturation can be maintained by this kind of compensation, diagnosis of hypoxia and the management of the underlying disease is delayed. In addition prolonged fast and deep breathing may be responsible for patient self-inflicted lung injury (P-SLI).

The respiratory monitoring described above can help identify this condition. As the tidal volume is estimated continuously, the (rolling) average volume of gas inhaled (or exhaled) per minute can be calculated. If this volume is above some predetermined threshold (e.g. 7l/min), an alert may be generated to further evaluate whether the increased breathing effort is for correction of a developing hypoxia and appropriately acted upon.

As discussed above, the system makes use of processor to perform the data processing. The processor can be implemented in numerous ways, with software and/or hardware, to perform the various functions required. The processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

**Claims**

1. A system (10) for remote monitoring of respiration of subject, comprising:

   an imaging system (20) comprising an imaging sensor (22) and a time of flight depth sensor (24); and
   a processor (26) for processing the image sensor signals and the depth sensor signals, wherein the processor is adapted to:

      receive data comprising images and depth information in respect of a field of view;
      process at least part of the data to detect a subject within the field of view;
      process at least part of the data to determine a body posture of the subject;
      determine when the subject body posture is suitable for the measurement of respiratory parameters, and
      when it is determined that a subject body posture is suitable for the measurement of respiratory parameters, measure respiratory parameters indicative of respiratory effort using temporal analysis of the images and depth information in respect of a chest area of the subject.

2. The system of claim 1, wherein the processor (26) is adapted to detect a front-facing subject orientation with respect to the imaging system.

3. The system of claim 1 or 2, wherein the processor (26) is adapted to identify anatomical landmarks of the chest.

4. The system of claim 3, wherein the processor (26) is adapted to collect depth data for analysis of chest wall dynamics.

5. The system of claim 4, wherein the processor (26) is adapted to determine a tidal volume for different chest compartments.

6. The system of claim 5, wherein the processor (26) is adapted to determine a tidal volume based on temporal amplitude and phase differences between time of flight measurements for different chest compartments.

7. The system of any one of claims 1 to 6, wherein the processor (26) is adapted to perform activity detection to detect a level of activity of the subject.

8. The system of any one of claims 1 to 7, wherein the imaging system (20) comprises a plurality of types of time of flight sensor.

9. The system of claim 8, wherein the imaging system (20) comprises a RGB sensor (22) for use in detecting a subject within the field of view and determining the body posture and a radar or ultrasound radar sensor for use in identifying and tracking anatomical landmarks of the chest.

10. The system of any one of claims 1 to 9, wherein the processor (26) is adapted to choose different sensor types based on a distance to the subject.

11. The system of any one of claims 1 to 10, wherein the processor (26) is adapted to derive a respiratory minute volume from a continuously estimated tidal volume.

12. A method for remote monitoring of respiration of subject, comprising:

    (30, 42) receiving data comprising images and depth information in respect of a field of view;
    (32) processing at least part of the data to detect a subject within the field of view;
    (34) processing at least part of the data to determine a body posture of the subject;
    (36) determining when a subject body posture is suitable for the measurement of respiratory parameters, and

    when it is determined that the subject body posture is suitable for the measurement of respiratory parameters, measuring (48) respiratory parameters indicative of respiratory effort using temporal analysis of the images and depth information in respect of a chest area of the subject.

13. The method of claim 12, comprising:

    detecting a front-facing subject orientation with respect to the imaging system.
    identifying anatomical landmarks of the chest;
    collecting depth data for analysis of chest wall dynamics; and
    determining a tidal volume for different chest compartments.

14. The method of claim 12 or 13, comprising performing activity detection to detect a level of activity of the subject and measuring respiratory parameters only for activity levels meeting activity level criteria.

15. A computer program comprising computer program code means which is adapted, when said program is run on a computer, to implement the method of any one of claims 12 to 14.

FIG. 1

FIG. 2

FIG. 3

Europäisches
Patentamt

European
Patent Office

Office européen
des brevets

# EUROPEAN SEARCH REPORT

Application Number

**EP 21 21 2800**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2019/209046 A1 (ADDISON PAUL S [GB] ET AL) 11 July 2019 (2019-07-11) | 1-4, 7-12,14, 15 | INV. A61B5/113 A61B5/091 |
| Y | * paragraphs [0081] - [0084] * <br> * paragraphs [0094] - [0107] * <br> * paragraphs [0117] - [0133] * <br> * paragraph [0150] * <br> * figures * | 5,6,13 | ADD. A61B5/08 A61B5/11 A61B5/0507 |
| Y | FERRIGNO G ET AL: "PRINCIPAL COMPONENT ANALYSIS OF CHEST WALL MOVEMENT IN SELECTED PATHOLOGIES", MEDICAL AND BIOLOGICAL ENGINEERING AND COMPUTING, SPRINGER, HEILDELBERG, DE, vol. 36, no. 4, 1 July 1998 (1998-07-01), pages 445-451, XP000755444, ISSN: 0140-0118 * page 445 * * left column; page 447 * * left column; page 449 * | 5,6,13 | |
| A | WO 2021/083577 A1 (CENTRE HOSPITALIER REGIONAL ET UNIV DE BREST [FR] ET AL.) 6 May 2021 (2021-05-06) * the whole document * | 1-15 | TECHNICAL FIELDS SEARCHED (IPC) A61B |
| A | US 2019/336004 A1 (MIHAILESCU LUCIAN [US] ET AL) 7 November 2019 (2019-11-07) * paragraphs [0077] - [0084] * | 8-10 | |

The present search report has been drawn up for all claims

| Place of search | Date of completion of the search | Examiner |
|---|---|---|
| The Hague | 16 May 2022 | Bataille, Frédéric |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 21 2800

This annex lists the patent family members relating to the patent documents cited in the above-mentioned European search report.
The members are as contained in the European Patent Office EDP file on
The European Patent Office is in no way liable for these particulars which are merely given for the purpose of information.

16-05-2022

| Patent document cited in search report | | Publication date | Patent family member(s) | | Publication date |
|---|---|---|---|---|---|
| US 2019209046 | A1 | 11-07-2019 | AU | 2018400475 A1 | 16-07-2020 |
| | | | CA | 3086527 A1 | 11-07-2019 |
| | | | CN | 111565638 A | 21-08-2020 |
| | | | EP | 3713486 A1 | 30-09-2020 |
| | | | US | 2019209046 A1 | 11-07-2019 |
| | | | WO | 2019135877 A1 | 11-07-2019 |
| WO 2021083577 | A1 | 06-05-2021 | NONE | | |
| US 2019336004 | A1 | 07-11-2019 | CA | 2866370 A1 | 12-09-2013 |
| | | | CN | 104271046 A | 07-01-2015 |
| | | | CN | 108095761 A | 01-06-2018 |
| | | | CN | 113974689 A | 28-01-2022 |
| | | | EP | 2822472 A1 | 14-01-2015 |
| | | | US | 2013237811 A1 | 12-09-2013 |
| | | | US | 2016242744 A1 | 25-08-2016 |
| | | | US | 2019336004 A1 | 07-11-2019 |
| | | | WO | 2013134559 A1 | 12-09-2013 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **AGOSTONI E ; MOGNONI P.** Deformation of the chest wall during breathing efforts. *J. Appl. Physiol.,* 21 November 1966, (6), 1827-32
- **KE SUN ; BIN XIAO ; DONG LIU ; JINGDONG WANGDEEP.** High-Resolution Representation Learning for Human Pose Estimation. *CVPR,* 2019
- **AOKI, H. ; MIYAZAKI, M. ; NAKAMURA, H. ; FURUKAWA, R. ; SAGAWA, R. ; KAWASAKI, H.** Non-contact respiration measurement using structured light 3-D sensor. *SICE Annual Conference (SICE) (Akita),* 2012